(19) Europäisches Patentamt
European Patent Office
Office européen des brevets

(11) EP 3 601 206 B1

(12) # EUROPÄISCHE PATENTSCHRIFT

(45) Veröffentlichungstag und Bekanntmachung des Hinweises auf die Patenterteilung:
**21.07.2021 Patentblatt 2021/29**

(21) Anmeldenummer: **18707929.8**

(22) Anmeldetag: **06.03.2018**

(51) Int Cl.:
***C07C 51/235*** (2006.01) ***C07C 59/58*** (2006.01)
***C09K 5/20*** (2006.01) ***C23F 11/12*** (2006.01)

(86) Internationale Anmeldenummer:
**PCT/EP2018/055443**

(87) Internationale Veröffentlichungsnummer:
**WO 2018/172062 (27.09.2018 Gazette 2018/39)**

(54) **VERFAHREN ZUR HERSTELLUNG EINER KORROSIONSSCHUTZKOMPONENTE FÜR EIN GEFRIERSCHUTZMITTEL**

PROCESS FOR PREPARING AN ANTICORROSION COMPONENT FOR AN ANTIFREEZE

PROCÉDÉ DE FABRICATION D’UN COMPOSANT DE PROTECTION CONTRE LA CORROSION POUR UN FLUIDE ANTIGEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.03.2017 EP 17161965**

(43) Veröffentlichungstag der Anmeldung:
**05.02.2020 Patentblatt 2020/06**

(73) Patentinhaber: **BASF SE**
**67056 Ludwigshafen am Rhein (DE)**

(72) Erfinder:
- **VAUTRAVERS, Nicolas**
  **67056 Ludwigshafen (DE)**
- **TELES, Joaquim Henrique**
  **67056 Ludwigshafen (DE)**
- **ALTHOEFER, Henning**
  **67056 Ludwigshafen (DE)**
- **DIETL, Harald**
  **67056 Ludwigshafen (DE)**

(74) Vertreter: **BASF IP Association**
**BASF SE**
**G-FLP-C006**
**67056 Ludwigshafen (DE)**

(56) Entgegenhaltungen:
**US-A- 4 256 916**

- **ZHANG YANYAN ET AL: "Preparation of diglycolic acid via oxidation of diethylene glycol with molecular oxygen", JINGXI HUAGONG - FINE CHEMICALS, ZHONGGUO HUAGONG XUEHUI, DALIAN, CN, Bd. 29, Nr. 5, 1. Januar 2012 (2012-01-01) , Seiten 517-520, XP009505502, ISSN: 1003-5214 -& DATABASE CA [Online] CHEMICAL ABSTRACTS SERVICE, COLUMBUS, OHIO, US; ZHANG, YANYAN ET AL: "Preparation of diglycolic acid via oxidation of diethylene glycol with molecular oxygen", XP002781224, gefunden im STN Database accession no. 2012:1169993**
- **YAIR EIN-ELI: "Enhanced Corrosion Inhibition of Zn in Alkaline Solutions Containing Poly(ethylene glycol) Diacid", ELECTROCHEMICAL AND SOLID-STATE LETTERS., Bd. 7, Nr. 1, 1. Januar 2004 (2004-01-01), Seiten B5-B7, XP55400886, US ISSN: 1099-0062, DOI: 10.1149/1.1626111**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 601 206 B1

## Beschreibung

Die vorliegende Erfindung betrifft ein Verfahren zur Herstellung einer Korrosionsschutzkomponente für ein Gefrierschutzmittel durch Oxidation eines Oxydiols der allgemeinen Formel (I)

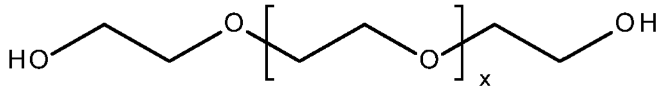

(I),

in der x für eine positive ganze Zahl von 1 bis 10 steht, mit molekularem Sauerstoff bei einer Temperatur von 20 bis 100° C und einem Sauerstoff-Partialdruck von 0,01 bis 2 MPa in Gegenwart von Wasser und eines heterogenen Katalysators, welcher Platin enthält, unter Bildung einer Oxydicarbonsäure der allgemeinen Formel (II)

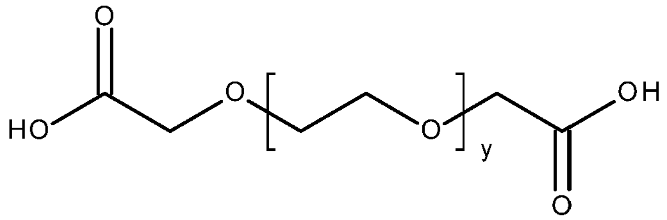

(II),

in der y für eine positive ganze Zahl von 1 bis 10 steht.

Gefrierschutzmittel werden vor allem in Kühlkreisläufen von Verbrennungsmotoren eingesetzt um darin ein Ausfrieren der Kühlmittelflüssigkeit bei niedrigen Temperaturen zu verhindern. Das Gefrierschutzmittel wird dabei dem eigentlichen Kühlmedium, bei dem es sich in der Regel um Wasser handelt, in der erforderlichen Menge zugesetzt.

Hauptbestandteil der Gefrierschutzmittel sind üblicherweise niedermolekulare Alkylenglykole wie Ethylenglykol und Propylenglykol. Gleichzeitig sollen Gefrierschutzmittel aber auch den Kühlkreislauf vor Korrosion schützen. Daher sind den Gefrierschutzmitteln in der Regel Korrosionsschutzmittel beigefügt. Bei diesen handelt es sich zumeist um eine Mischung aus verschiedenen Substanzen aus verschiedenen chemischen Stoffklassen. Eine wichtige Stoffklasse bei den eingesetzten Korrosionsschutzmittel stellen mittel- bis langkettige Carbonsäuren dar. Bei diesen handelt es sich üblicherweise um gesättigte und ungesättigte, verzweigte und unverzweigte, aliphatische und aromatische Mono- und Dicarbonsäuren mit gewöhnlich 3 bis 16 Kohlenstoffatomen.

EP 0,479,470 B1 offenbart eine Gefrierschutzmittelzusammensetzung, in der neben weiteren Komponenten als Korrosionsschutzmittel Neopentansäure, Isononansäure, Neoheptansäure, Dimethylglutarsäure, Diethylmalonsäure, 2-Ethylbuttersäure, Methylvaleriansäure, Korksäure, Azelainsäure, Sebacinsäure, Undecandisäure, Dodecandisäure, Dicyclopentadiendisäure oder Terephtalsäure eingesetzt werden. Sebacinsäure (Decandisäure) ist als besonders bevorzugt genannt.

In EP 0,552,988 B1 ist eine Gefrierschutzmittelzusammensetzung beschrieben, in der neben weiteren Komponenten als Korrosionsschutzmittel Malonsäure, Bernsteinsäure, Glutarsäure, Adipinsäure, Pimelinsäure, Sebacinsäure, Azelainsäure, Itaconsäure oder Heptansäure eingesetzt werden. Als besonders bevorzugt sind Sebacinsäure und Azelainsäure (Nonandisäure) genannt.

US 4,561,990 lehrt als Korrosionsschutzmittel den Einsatz einer Mischung aus einem Alkali-Molybdat und einer $C_{8-12}$-Dicarbonsäure wie Korksäure, Azelainsäure, Sebacinsäure, Undecandisäure oder Dodecandisäure, wobei auch hier Sebacinsäure als besonders bevorzugt hervorgehoben wurde.

EP 0,229,440 B1 nennt eine Gefrierschutzmittelzusammensetzung, in der neben weiteren Komponenten als Korrosionsschutzmittel Octansäure, Nonansäure, Decansäure, Undecansäure, Dodecansäure, Korksäure, Azelainsäure, Sebacinsäure, Undecandisäure, Dodecandisäure, Dicyclopentadiendisäure oder Terephtalsäure eingesetzt werden. Auch bei dieser Aufzählung wurde Sebacinsäure als besonders bevorzugt erwähnt.

Die im oben zitierten Stand der Technik genannten Mono- und Dicarbonsäuren weisen zwar eine gute Korrosionsschutzwirkung auf, sind jedoch aufgrund ihrer chemischen Struktur teilweise nur aufwendig in größeren Mengen herstellbar. Zudem zeigen insbesondere die langkettigen Mono- und Dicarbonsäuren eine geringe Löslichkeit in polaren Medien wie Wasser. So besitzt beispielsweise Sebacinsäure in Wasser bei 20° C nur eine Löslichkeit von ca. 1 g/l.

Y. Ein-Eli, Electrochemical and Solid-State Letters, 7 (1) B5-B7, 2004, fand durch Versuche an reinem Zn-Metall heraus, dass Oxycarbonsäuren im Vergleich zu Polyethylenglykol eine bessere Korrosionsinhibierung aufweisen und sowohl den Korrosionsstrom erheblich reduzieren als auch das Inhibierungspotenzial zu sehr niedrigen Potenzialwerten verschieben.

Im Zuge der Suche nach einem verbesserten Gefrierschutz- und Korrosionsschutzmittel wurde nun ein Konzentrat mit verbesserten Eigenschaften gefunden.

Dieses ist nicht Teil der beanspruchten Erfindung und enthält

(1) 1 bis 10 Gew.-% eines Gemisches aus

30-100 Gew.-%

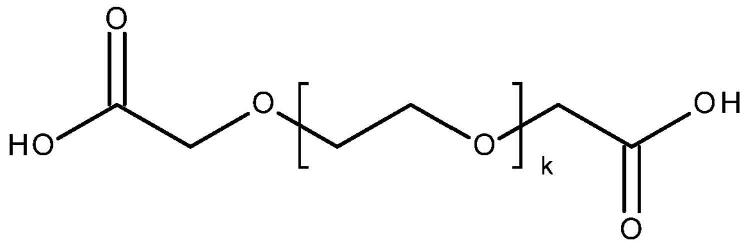

,

0-40 Gew.-%

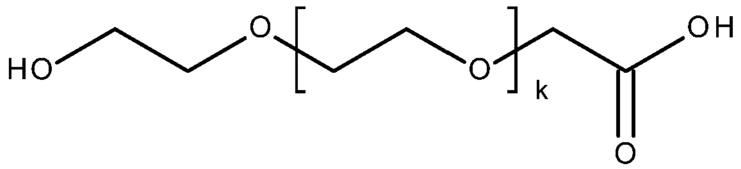

,

und

0-30 Gew.-%

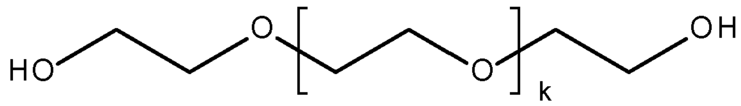

,

wobei k unabhängig voneinander jeweils für 0 (null) oder eine positive ganze Zahl von 1 bis 10 steht, sowie die Summe der Mengen der drei Komponenten 100 Gew.-% ergibt, und

(2) 90 bis 99 Gew.-% weiterer Komponenten.

Bei den genannten weiteren Komponenten kann es sich um Additive aus einer oder mehrerer Substanzklassen handeln. Bei diesen kann es sich beispielsweise um einwertige, zweiwertige oder dreiwertige Alkohole, Polyhydroxyalkohole oder deren Ether handeln. Als Beispiele derartiger Alkohole seien Ethylenglykol, 1,2-Propylenglykol (1,2-Propandiol), Diethylenglykol, Dipropylenglykol, Triethylenglykol, Tetraethylenglykol, Pentaethylenglykol, Hexaethylenglykol, Dipropylenglykol, Tripropylenglykol, Tetrapropylenglykol, Pentapropylenglykol, Hexapropylenglykol, 1,3-Propylenglykol, Glycerin, Monoether von Glykolen wie der Methyl-, Ethyl-, Propyl- und Butylether von Ethylenglykol, Propylenglykol, Diethylenglykol und Dipropylenglykol genannt. Bevorzugt sind dabei Ethylenglykol, Propylenglykol und Glycerin, insbesondere Ethylenglykol.

Als weitere, mögliche Additive im Konzentrat seien genannt

(a) aliphatische, cycloaliphatische oder aromatische Monocarbonsäuren mit jeweils 3 bis 16 C-Atomen in Form von deren Alkalimetall-, Ammonium- oder substituierten Ammoniumsalzen;

(b) aliphatische oder aromatische Di- oder Tri-carbonsäuren mit jeweils 3 bis 21 C-Atomen in Form von deren Alkalimetall-, Ammonium- oder substituierter Ammoniumsalzen;

(c) Alkalimetallborate, Alkalimetallphosphate, Alkalimetallsilikate, Alkalimetallnitrite, Alkali- oder Erdalkalimetallnitrate, Alkalimetallmolybdate oder Alkali- oder Erdalkalimetallfluoride;

(d) aliphatische, cycloaliphatische oder aromatische Amine mit 2 bis 15 C-Atomen, welche zusätzlich Ethersauerstoffatome oder Hydroxylgruppen enthalten können;

(e) ein- oder zweikernige ungesättigte oder teilungesättigte Heterocyclen mit 4 bis 10 C-Atomen, welche benzanelliert sein können und/oder zusätzliche funktionelle Gruppen tragen können;

(f) Tetra-($C_1$-$C_8$-alkoxy)-silane (Orthokieselsäuretetra-$C_1$-$C_8$-alkylester);

(g) Carbonsäure- oder Sulfonsäureamide;

(h) Hartwasserstabilisatoren auf Basis von Polyacrylsäure, Polymaleinsäure, Acrylsäure-Maleinsäure-Copolymeren, Polyvinylpyrrolidon, Polyvinylimidazol, Vinylpyrrolidon-Vinylimidazol-Copolymeren und/oder Copolymeren aus ungesättigten Carbonsäuren und Olefinen.

Zur Herstellung eines gebrauchsfertigen Gefrierschutzmittels ist das gefundene Konzentrat mit einem Basis-Gefrierschutzmittel zu verdünnen, welches dann den Hauptbestandteil des gebrauchsfertigen Gefrierschutzmittels darstellt. Wie eingangs erwähnt, kommen auch im vorliegenden Fall als Basis-Gefrierschutzmittel vor allem niedermolekulare Alkylenglykole wie Ethylenglykol und Propylenglykol zum Einsatz.

Eine wesentliche Komponente des gefundenen Korrosionsschutzmittelkonzentrats, die nicht Teil der beanspruchten Erfindung ist, ist die Oxydicarbonsäure

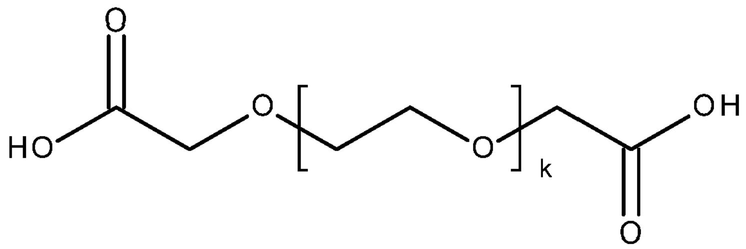

,

in der k die oben genannte Bedeutung hat. Oxydicarbonsäuren können beispielsweise durch Oxidation der $-CH_2OH$-Gruppen der entsprechenden Oxydiole hergestellt werden. Die entsprechenden Oxydiole sind großtechnisch durch Polymerisation von Ethylenoxid in Gegenwart von Wasser oder durch Ethoxylierung von Ethylenglykol mit Ethylenoxid relativ einfach zugänglich.

Eine gängige Methode zur Oxidation von $-CH_2OH$-Gruppen zu den entsprechenden -COOH-Gruppen ist die heterogenkatalytische Oxidation mit Sauerstoff in wässriger Flüssigphase in Gegenwart eines Pt-haltigen Katalysators. So wurde in K.Heyns et al., Tetrahedron, 1960, Vol. 9, 67-75 die Pt-katalysierte Oxidation von primären und sekundären Hydroxylverbindungen zu den entsprechenden Aldehyden, Ketonen und Carbonsäuren untersucht. Darin wurde festgestellt, dass in neutraler Lösung primäre Alkohole im Wesentlichen nur zu den Aldehyden oxidiert werden und die durch teilweise Weiteroxidation der Aldehyde gebildeten geringen Mengen an Säure die weitere Oxidation hemmen, so dass die Ausbeuten gering sind. Erst durch Zugabe von mindestens stöchiometrischen Mengen an Alkali, wie beispielsweise NaOH, konnten gute Ausbeuten an Carbonsäuren erreicht werden.

C.Donze et al., Applied Catalysis B: Environmental 70 (2007) 621-629 bestätigt diese Erkenntnis am Beispiel der Pt-katalysierten Oxidation von Benzylalkohol. Ohne Zugabe von NaOH wurden selbst nach mehreren Stunden Reaktionszeit nur geringe Mengen an Benzoesäure erhalten, wohingegen die Selektivität zum Benzaldehyd sehr hoch war. Erst in Gegenwart einer mindestens stöchiometrischen Menge an NaOH konnte die Benzoesäure in hoher Ausbeute erhalten werden.

H.Fiege et al., Angew. Chem. 93 (1981) Nr. 9, 812-813 sowie US 4,238,625 zeigen, dass selbst in Gegenwart einer mindestens stöchiometrischen Menge an NaOH an einem Katalysator mit 1% Pt auf Aktivkohle keine Oxidation von 2-Aryloxyethanol erfolgt. Erst durch Bereitstellung eines durch Pb, Bi und/oder Cd aktivierten Pt-haltigen Katalysators konnte eine Oxidation zu 2-Aryloxyessigsäuren ermöglicht werden.

DE 31 35 946 A1 lehrt die Oxidation von 2-Alkyloxyethanolen in einem wässrig-alkalischen Medium mit Sauerstoff in Gegenwart eines, mit Pb, Bi und/oder Cd aktivierten Pt-haltigen Katalysators zu den entsprechenden 2-Alkyloxyessigsäuren. Auch diese Schrift zeigt durch Kontrollexperimente, dass selbst in Gegenwart einer mindestens stöchiometrischen Menge an NaOH an einem nicht durch Pb, Bi und/oder Cd aktivierten Katalysator mit 1% Pt auf Aktivkohle keine Oxidation des 2-Alkyloxyethanols erfolgt. Erst durch Bereitstellung eines durch Pb, Bi und/oder Cd aktivierten Pt-haltigen Katalysators konnte eine Oxidation zur 2-Alkyloxyessigsäure ermöglicht werden. Für die Bereitstellung eines wässrig-alkalischen Mediums sind als geeignete Alkalien alkalische Verbindungen von Alkali- und Erdalkalimetallen wie beispielsweise die Hydroxide und Carbonate von Na und K genannt. Da diese stöchiometrisch durch die gebildete Alkyloxyessigsäure gebunden werden, sind sie entsprechend der Lehre von DE 31 35 846 A1 im Überschuss in einer Menge von 1 bis 1,5 mol Alkali pro Mol Alkyloxyethanol einzusetzen.

Nachteilig an den oben genannten Oxidationsverfahren in Gegenwart eines wässrig-alkalischen Mediums ist die Bildung des entsprechenden Carbonsäuresalzes als direktes Oxidationsprodukt. Die freie Carbonsäure ist daraus erst in einem Folgeschritt durch Umsetzung mit einer Säure und Isolierung aus der angesäuerten Lösung erhältlich. Dieses Prozedere ist nicht nur sehr aufwendig, sondern erfordert zudem einen mindestens stöchiometrischen Einsatz einer Base und

nachfolgend einen mindestens stöchiometrischen Einsatz einer Säure. Somit wird eine mindestens stöchiometrische Menge an Salz produziert, welches abzutrennen und zu entsorgen ist.

In DE 29 36 123 A wurde zumindest für die Oxidation von 2-Alkyloxyethanolen erkannt, dass die Oxidation an Pt-haltigen Katalysatoren auch ohne Zusatz einer Base zur Bildung von 2-Alkyloxyessigsäuren führt. So wurde in Beispiel 1 bei der Oxidation von Methylglykol an 5% Pt auf Aktivkohle bei einem Molverhältnis von Pt zu Methylglykol von 0,0065 und einer Reaktionstemperatur von 45° C bei Normaldruck eine Ausbeute an Methoxyessigsäure von 95% erreicht. Allerdings wurden bei der Oxidation höhermolekularer Alkoxyessigsäuren, wie n-Butylglykol und Methyldiglykol, trotz eines höheren Molverhältnisses von Pt zum entsprechenden 2-Alkyloxyethanol von 0,01 unter sonst gleichen Reaktionsbedingungen deutlich geringere Ausbeuten als beim niedermolekularen Methylglykol erhalten. So betrug die Ausbeute an n-Butoxyessigsäure nur 90% und jene an Methoxyethoxyessigsäure nur 91%.

US 4,256,916 lehrt die Oxidation von Polyethylenglykol mit Sauerstoff in der Flüssigphase ohne Zugabe einer Base und in Gegenwart von Platin auf Aktivkohle in einem Festbettreaktor zu den entsprechenden Polyethylenglykoldisäuren, wobei die Polyethylenglykol-haltige Eduktlösung mit einer Geschwindigkeit von 0,1 bis 0,6 Fuß pro Sekunde (3,05 bis 18,3 cm pro Sekunde) über den Festbettkatalysator fließt. In den Beispielen wurde jeweils eine wässrige Lösung von Diethylenglykol beziehungsweise Triethylenglykol in einen Rohrreaktor mit einem Pt/Aktivkohle Festbettkatalysator gegeben und unter kontinuierlicher Zufuhr von Sauerstoff im Kreis gepumpt, so dass die Strömungsgeschwindigkeit über dem Katalysator im Bereich 0,1 bis 0,4 Fuß pro Sekunde (3,05 bis 12,2 cm pro Sekunde) betrug. Das anfängliche Molverhältnis von Pt zum eingesetzten Polyethylenglykol (Diethylenglykol beziehungsweise Triethylenglykol) lag im Bereich von 0,02 bis 0,031.

Durch die Einstellung einer hohen Strömungsgeschwindigkeit über den Festbettkatalysator konnte zwar ein Polyethylenglykol-Umsatz von bis zu 99% erreicht werden, jedoch betrug die Ausbeute an der entsprechenden Polyethylenglykolsäure nur 49 bis 90,7%. Somit wurde ein nicht unerheblicher Teil des Edukts zu unerwünschten Nebenprodukten umgesetzt. Wesentliches Nebenprodukt war Glykolsäure (Hydroxyessigsäure). Glykolsäure entsteht durch oxidativen Abbau von -$CH_2$-O-$CH_2CH_2$OH Gruppen. Die Reaktionsgleichung am Beispiel von Diethylenglykol lautet:

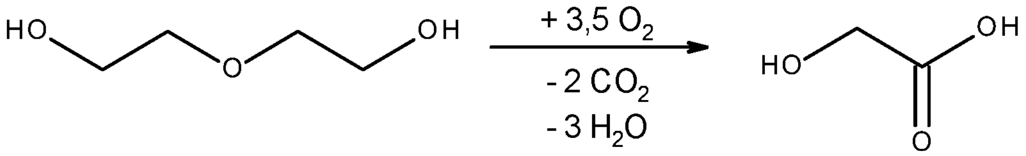


In den Beispielen der US 4,256,916 wurde Glykolsäure in einem sehr hohen Verhältnis von 2,9 bis 11,7 g/100 g Diglykolsäure erhalten.

Der oxidative Abbau zur Glykolsäure mindert nicht nur die Ausbeute an der gewünschten Diglykolsäure, sondern führt auch zu einem Verlust an wertvollen $CH_2$-Einheiten.

Im Rahmen der vorliegenden Erfindung wurde erkannt, dass Glykolsäure als relativ starke und kurzkettige Säure mit einem pKs-Wert von 3,83 die Korrosion eher fördert als mindert und somit in einer Menge, wie sie nach dem Verfahren der US 4,256,916 gebildet wird, als Bestandteil in einem Gefrierschutz- und Korrosionsschutzmittel ungeeignet ist. Würde man daher eine nach dem Verfahren der US 4,256,916 hergestellte Oxydicarbonsäure (Polyglykoldisäure) als Korrosionsschutzkomponente für ein Gefrierschutzmittel einsetzen wollen, so wäre eine vorherige Abtrennung des Großteils der Glykolsäure erforderlich. Eine Abtrennung der Glykolsäure aus einem wässrigen Polyglykoldisäure-Gemisch ist jedoch außerordentlich schwierig. Bei dem Versuch einer destillativen Abtrennung würden neben der Glykolsäure zwangsläufig auch größere Mengen an Wasser abgetrennt werden. Dies führt nicht nur zu einem deutlich erhöhten Energieaufwand, sondern auch dazu, dass das entwässerte Polyglykoldisäure-Gemisch im Kolonnensumpf aufgrund des hohen Schmelzpunktes als Feststoff ausfällt. Um das Polyglykoldisäure-Gemisch im Kolonnensumpf flüssig zu halten, müsste stets Wasser zugefügt werden, welches den Energieaufwand noch mehr in die Höhe treibt. Eine Abtrennung der Glykolsäure aus dem wässrigen Polyglykoldisäure-Gemisch ist daher aufgrund der äußerst schlechten Energiebilanz wirtschaftlich nicht sinnvoll. Somit ist das in US 4,256,916 beschriebene Verfahren trotz Verwendung von Polyethylenglykol als leicht zugänglichen Einsatzstoff für die Herstellung des gewünschten Gefrierschutz- und Korrosionsschutzmittel-Konzentrats nicht brauchbar.

Aufgabe der vorliegenden Erfindung war es daher, ein Verfahren zur Herstellung einer, für den Einsatz als Korrosionsschutzkomponente für ein Gefrierschutzmittel geeigneten Oxydicarbonsäure zu finden, welches auf den Einsatz eines gut verfügbaren Einsatzstoffes beruht, einfach durchzuführen ist, eine hohe Ausbeute und Reinheit ermöglicht, und vor allem ein Reaktionsprodukt erzeugt, welches auch ohne aufwendige Aufreinigung als Korrosionsschutzkomponente für ein Gefrierschutzmittel einsetzbar ist. In diesem Zusammenhang soll das Reaktionsprodukt keine oder nur in geringfügiger Menge Komponenten enthalten, welche der Wirkung als Korrosionsschutzkomponente für ein Gefrierschutzmittel entgegenstehen. Explizit soll daher der Gehalt an Glykolsäure relativ niedrig sein.

Überraschend wurde ein Verfahren zur Herstellung einer Korrosionsschutzkomponente für ein Gefrierschutzmittel durch Oxidation eines Oxydiols der allgemeinen Formel (I)

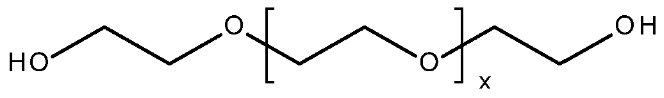

(I),

in der x für eine positive ganze Zahl von 1 bis 10 steht, mit molekularem Sauerstoff bei einer Temperatur von 20 bis 100° C und einem Sauerstoff-Partialdruck von 0,01 bis 2 MPa in Gegenwart von Wasser und eines heterogenen Katalysators, welcher Platin enthält, unter Bildung einer Oxydicarbonsäure der allgemeinen Formel (II)

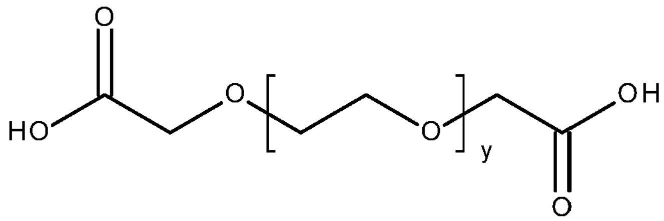

(II),

in der y für eine positive ganze Zahl von 1 bis 10 steht, gefunden, bei dem man die Oxidation

(a) bei einem Molverhältnis von

$$0{,}002 \leq \; n(Pt) \,/\, [n(Oxydiol\ (I)) + n(Oxydicarbonsäure\ (II))] \; \leq 0{,}019$$

wobei "n(Pt)" für die Molmenge an Platin, "n(Oxydiol (I))" für die Molmenge an Oxydiol (I) und "n(Oxydicarbonsäure (II))" für die Molmenge an Oxydicarbonsäure (II) steht;

(b) bei einer Konzentration an Wasser von 50 bis 95 Gew.-% in der flüssigen Phase; und

(c) bei einem pH-Wert von 1 bis 7

durchführt.

Ausgangsstoff für die Herstellung der genannten Korrosionsschutzkomponente ist ein Oxydiol der allgemeinen Formel (I)

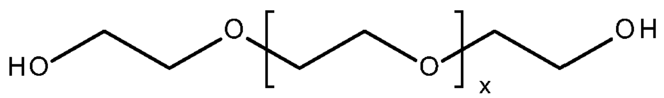

(I),

in der x für eine positive ganze Zahl von 1 bis 10 steht. Bevorzugt steht x für eine positive ganze Zahl von 1 bis 8, besonders bevorzugt von 1 bis 6, ganz besonders bevorzugt von 1 bis 5 und insbesondere von 1 bis 4.

Wie eingangs bereits erwähnt, sind Oxydiole (I) großtechnisch relativ einfach durch Polymerisation von Ethylenoxid in Gegenwart von Wasser oder durch Ethoxylierung von Ethylenglykol mit Ethylenoxid zugänglich. Oxydiole (I) mit einer mittleren Molmasse von 200 bis 400 g/mol sind bei Raumtemperatur flüssig. Da sie hygroskopisch sind, enthalten sie oftmals geringe Mengen an Wasser.

Bei dem einzusetzenden Oxydiol (I) kann es sich um eine konkrete Spezies mit einem bestimmten Wert für x oder um ein Gemisch aus Oxydiolen (I) mit unterschiedlichen Werten für x handeln. Da Oxydiole (I) herstellungsbedingt bereits als Gemische verschiedener Oxydiole (I) mit unterschiedlichen Werten für x anfallen, werden bevorzugt auch entsprechende Gemische mit unterschiedlichen Werten für x eingesetzt. Diese werden in der Praxis oftmals durch die Buchstabenkombination "PEG" für Polyethylenglykol und nachfolgender Zahl, die die mittlere Molmasse angibt, bezeichnet. So steht beispielsweise "PEG 200" für Polyethylenglykol mit einer mittleren Molmasse von 200 g/mol.

Somit werden bevorzugt Gemisch von Oxydiolen (I) mit einer mittlere Molmasse von 125 bis 500 g/mol, besonders bevorzugt von ≥ 140 g/mol und ganz besonders bevorzugt von ≥ 150 g/mol, sowie besonders bevorzugt von ≤ 400 g/mol und ganz besonders bevorzugt von ≤ 300 g/mol eingesetzt. Die mittlere Molmasse des Oxydiols (I) ist definiert

als Quotient aus der Summe der Massen der verschiedenen Oxydiole (I) im Gemisch und der Summe der Stoffmengen der verschiedenen Oxydiole (I) im Gemisch. Experimentell lässt sich die mittlere Molmasse leicht über die Messung der Hydroxylzahl bestimmen, wobei sich daraus die mittlere Stoffmenge der Oxydiole (I) im Gemisch berechnen lässt.

Das beim erfindungsgemäßen Verfahren einzusetzende Edukt kann neben dem Oxydiol (I) noch weitere Komponenten, wie beispielsweise Wasser, enthalten. In der Regel weisen die Oxydiole (I) herstellungsbedingt jedoch eine relativ hohe Reinheit auf.

Das Oxydiol (I) wird beim erfindungsgemäßen Verfahren zur Oxydicarbonsäure der allgemeinen Formel (II)

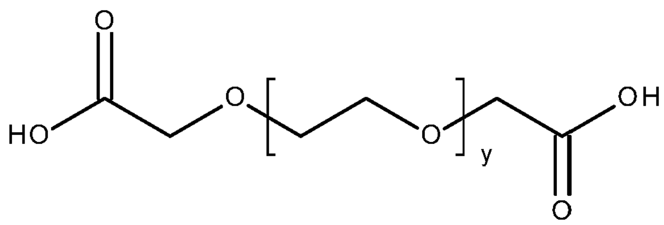

(II),

oxidiert, wobei y ebenfalls für eine positive ganze Zahl von 1 bis 10 steht. Bevorzugt steht y für eine positive ganze Zahl von 1 bis 8, besonders bevorzugt von 1 bis 6, ganz besonders bevorzugt von 1 bis 5 und insbesondere von 1 bis 4.

Wie bei dem eingesetzten Oxydiol (I) kann es sich auch bei der gebildeten Oxydicarbonsäure (II) um eine konkrete Spezies oder um ein Gemisch verschiedener Oxydicarbonsäure (II) handeln. Wird ein Gemisch verschiedener Oxydiol (I) eingesetzt, so wird auch ein Gemisch verschiedener Oxydicarbonsäure (II) erhalten.

Das erfindungsgemäße Verfahren wird in Gegenwart von Wasser durchgeführt. Wasser fördert in mehrfacher Hinsicht die Umsetzung vom Oxydiol (I) zur Oxydicarbonsäure (II). So verbessert Wasser im Falle des Einsatzes eines Suspensionskatalysators dessen Suspension im Reaktionsgemisch und setzt zudem auch die Viskosität des Reaktionsgemisches herab. Durch den Verdünnungseffekt wird der Wärmeeintrag durch die freiwerdende Oxidationswärme verringert und somit einer zu starken Erwärmung entgegengewirkt. Der wesentliche Vorteil durch den Einsatz von Wasser liegt jedoch in der physikalischen Natur der Oxydicarbonsäuren (II). Da diese einen deutlich höheren Schmelzpunkt als die entsprechenden Oxydiole (I) aufweisen, würden ohne Gegenwart von Wasser diese im Laufe der Oxidationsreaktion als Feststoff ausfallen und somit eine sichere Reaktionsführung mit hohem Umsatz, hoher Selektivität und leichter Aufarbeitung des Reaktionsgemisches unterbinden. So hat bereits die Diglykolsäure (Oxydicarbonsäuren (II) mit x=0) einen Schmelzpunkt von oberhalb 140° C. Aufgrund der sehr guten Wasserlöslichkeit werden die gebildeten Oxydicarbonsäuren (II) in Lösung gehalten.

Das erfindungsgemäße Verfahren erfolgt daher in wässriger Lösung in der Flüssigphase, wobei die Konzentration an Wasser in der Flüssigphase 50 bis 95 Gew.-% beträgt. Die Konzentration an Wasser in der Flüssigphase beträgt bevorzugt ≥ 60 Gew.-%, sowie bevorzugt ≤ 70 Gew.-%.

Als Katalysator setzt man beim erfindungsgemäßen Verfahren einen heterogenen Katalysator ein, welcher Platin als aktive Komponente enthält. Üblicherweise ist das Platin auf einem Träger fixiert. Als Träger können die verschiedensten Materialien eingesetzt werden. Als Beispiele seien anorganische Oxide wie etwa Aluminiumoxid, Zirkoniumoxid, Titandioxid, Siliziumoxid, anorganische Silikate wie etwa Aluminiumsilikat oder Kohle genannt. Es können natürlich auch Mischungen verschiedener Trägermaterialien eingesetzt werden. Bevorzugt ist der Einsatz von Kohle als Träger. Der Katalysator enthält im Allgemeinen 0,1 bis 10 Gew.-%, bevorzugt ≥ 0,5 Gew.-%, besonders bevorzugt ≥ 1 Gew.-% und ganz besonders bevorzugt ≥ 4 Gew.-%, sowie bevorzugt ≤ 8 Gew.-% und besonders bevorzugt ≤ 6 Gew.-% Platin, jeweils bezogen auf die Gesamtmasse des heterogenen Katalysators.

Besonders bevorzugt setzt man beim erfindungsgemäßen Verfahren einen heterogenen Katalysator enthaltend 0,1 bis 10 Gew.-%, ganz besonders bevorzugt enthaltend 1 bis 10 Gew.-% und insbesondere enthaltend 4 bis 10 Gew.-% Platin auf Kohle ein.

Der einzusetzende Katalysator kann neben Platin noch weitere Metalle enthalten. Unter dem Begriff "weitere Metalle" sind Metalle der vierten bis sechsten Periode der Gruppen 3 bis 16 des Periodensystems der Elemente, beginnend mit Scandium (Ordnungszahl 21) und endend mit Polonium (Ordnungszahl 84) zu verstehen. Sind noch weitere Metalle zugegen, so ist deren Gehalt vorteilhafterweise sehr niedrig. Vorzugsweise liegt der Gesamtgehalt weiterer Metalle bei 0 bis 5 Gew.-%, bevorzugt bei 0 bis 1 Gew.-%, besonders bevorzugt bei 0 bis 0,5 Gew.-%, ganz besonders bevorzugt bei 0 bis 0,1 Gew.-% und insbesondere bei 0 bis 0,01 Gew.-%, bezogen auf die Masse an Platin. Insbesondere liegt der Gesamtgehalt an Cadmium, Blei und Bismut bei vorzugsweise 0 bis 1 Gew.-%, bevorzugt bei 0 bis 0,5 Gew.-%, besonders bevorzugt bei 0 bis 0,1 Gew.-%, ganz besonders bevorzugt bei 0 bis 0,05 Gew.-% und insbesondere bei 0 bis 0,01 Gew.-%, bezogen auf die Masse an Platin. Die Herstellung des Katalysators erfolgt somit bevorzugt ohne gezielte Zugabe weiterer Metalle.

Der heterogene, geträgerte Katalysator kann in verschiedenen geometrischen Formen und Größen, wie etwa

als Pulver oder Formkörper, eingesetzt werden. Pulverförmige Katalysatoren können beispielsweise in Suspensionsfahrweise betrieben werden. Bei einer Festbettfahrweise setzt man bevorzugt Formkörper ein, wie beispielsweise Granulat, Zylinder, Hohlzylinder, Kugeln oder Extrudate. Die Formkörper sind dann üblicherweise nach den bekannten Methoden im Reaktor fixiert. Im Falle von Katalysatorformkörpern weisen diese bevorzugt eine mittlere Partikelgröße von 1 bis 10 mm auf.

Bevorzugt setzt man jedoch den Katalysator in Form eines Pulvers ein. Der pulverförmige Katalysator liegt dann im Reaktor in Suspension vor. Um einen Austrag aus dem Reaktionssystem zu verhindern, wird hierbei üblicherweise ein Filter zum Rückhalt des Suspensionskatalysators eingesetzt. Als Beispiel eines üblicherweise geeigneten Filters sei der Querstromfilter genannt.

Unabhängig von der geometrischen Form und Größe der Katalysatorpartikel liegt das Platin im Allgemeinen in Form von Partikeln mit einem mittleren Durchmesser von 0,1 bis 50 nm, gemessen über Röntgenbeugung, vor. Es können jedoch auch kleinere oder größere Partikel vorliegen.

Bei der Herstellung des heterogenen, geträgerten Katalysators wird das Platin im Allgemeinen durch geeignete Methoden auf den Träger aufgebracht.

Platin wird dabei üblicherweise aus Lösungen geeigneter Salze auf den Träger aufgebracht. Geeignete Platinsalze sind beispielsweise solche, welche in wässrigen beziehungsweise wässrig-sauren Medien löslich sind und aus denen durch eine Erhöhung des pH-Wertes eine Platinverbindung ausgefällt werden kann. Als bevorzugte Beispiele eines geeigneten Platinsalzes seien Platin(II)nitrat, Platin(IV)chlorid und Hexachloroplatinsäure Hexahydrat genannt. Als pH-Wert erhöhende Mittel kommen insbesondere wässrige Lösungen alkalischer Salze in Frage, wie beispielsweise Alkalicarbonate, bevorzugt Natriumcarbonat.

Zur Aufbringung der unlöslichen oder schwer löslichen Platinverbindungen sind prinzipiell die verschiedensten Methoden möglich. In einer bevorzugten Ausführungsform wird der Träger in einer geeigneten Apparatur, beispielsweise einer rotierenden Trommel oder einem gerührten Behälter, in überstehender Flüssigkeit, beispielsweise Wasser, vorgelegt und mit der Lösung des Platinsalzes sowie der pH-Wert erhöhenden Lösung versetzt. Dabei ist es möglich, zuerst das Platinsalz und anschließend die pH-Wert erhöhende Lösung, oder zuerst die pH-Wert erhöhende Lösung und anschließend das Platinsalz, oder beide alternierend oder auch gleichzeitig zuzufügen.

Bevorzugt wird der Träger in Wasser vorgelegt und der pH-Wert mit der pH-Wert erhöhenden Lösung auf einen Wert eingestellt, bei dem das Platinsalz als unlösliche oder schwer lösliche Platinverbindung ausfällt. Anschließend wird unter Durchmischung die Lösung des Platinsalzes zugefügt, wobei der pH-Wert durch weitere Zugabe der den pH-Wert erhöhenden Lösung in einem Bereich gehalten wird, in dem das Platinsalz als unlösliche oder schwer lösliche Platinverbindung ausfällt. Das Gewichtsverhältnis zwischen der insgesamt zuzugebenden Flüssigkeitsmenge und dem Träger liegt im Allgemeinen bei einem Wert von 1 bis 100.

Nach erfolgter Auffällung wird der, die Platinverbindung enthaltende Träger isoliert, getrocknet und reduziert. Als Reduktionsmittel werde im Allgemeinen die zur Reduktion von Edelmetallsalzen geeigneten Mittel eingesetzt. Beispielhaft genannt seien Wasserstoff, Alkohole wie etwa Ethanol oder Isopropanol, sowie Ascorbinsäure.

Bei der Imprägnierung wird die Lösung eines geeigneten Platinsalzes in einer geeigneten Apparatur, beispielsweise einer rotierenden Mischtrommel auf den Träger ausgesprüht. Die insgesamt aufzusprühende Menge an Platinsalz-Lösung liegt dabei bevorzugt bei oder unterhalb der Flüssigkeitsaufnahme des vorgelegten Trägers. Bei der Imprägnierung werden bevorzugt Platinsalze eingesetzt, welche sich durch Tempern rückstandsfrei in elementares Platin umwandeln. Bevorzugte Platinsalze zur Imprägnierung sind beispielsweise Platin(II)nitrat sowie Hexachloroplatinsäure.

Der heterogene, geträgerte Katalysator weist im Allgemeinen eine BET-Oberfläche von $\geq 1\ m^2/g$ und $\leq 10000\ m^2/g$, bestimmt nach DIN ISO 9277:2014-01, auf. Beim Einsatz von Kohle als Träger liegt die BET-Oberfläche bevorzugt im Bereich von $\geq 500\ m^2/g$ und $\leq 10000\ m^2/g$.

Es wurde nun überraschend gefunden, dass die bei der Oxidation eingesetzte Menge an Platin einen entscheidenden Einfluss auf die Menge an gebildeter Glykolsäure ausübt. Beim Einsatz einer hohen Menge an Platin in Bezug auf die vorliegende Gesamtmenge an Oxydiol (I) und Oxydicarbonsäure (II) wird unverhältnismäßig viel unerwünschte Glykolsäure gebildet. Mit sinkender Menge an Platin sinkt auch der Gehalt an Glykolsäure im Reaktionsprodukt, wobei die Reaktionsgeschwindigkeit zur Oxydicarbonsäure (II) über einen breiten Bereich ausreichend hoch bleibt. Erst bei einer sehr niedrigen Menge an Platin fällt die Reaktionsgeschwindigkeit auf einen unattraktiv geringen Wert ab.

Noch überraschender ist die Erkenntnis, dass die Ausbeute an Oxydicarbonsäure (II) mit sinkender Menge an Platin deutlich zunimmt. Eigentlich wäre genau das Gegenteil erwartet worden. So konnte beispielsweise durch eine Verringerung der Menge an Platin auf nur noch 40% des Ausgangswertes eine Steigerung der Ausbeute an Oxydicarbonsäure (II) um rund 65% auf somit 165% des Ausgangswertes erreicht werden.

Es wurde somit überraschend ein Bereich für das Verhältnis der Molmenge an Platin bezogen auf die Summe der Molmengen an Oxydiol (I) und Oxydicarbonsäure (II) gefunden, bei dem einerseits die Reaktionsgeschwindigkeit noch in einem effizienten Bereich liegt und andererseits die Menge an gebildeter Glykolsäure so niedrig ist, dass diese bei der bestimmungsgemäßen Verwendung des Reaktionsgemischs als Korrosionsschutzkomponente daraus nicht aufwendig abgetrennt werden muss. Das erfindungsgemäße Molverhältnis beträgt

$$0{,}002 \leq \ n(Pt) / [n(Oxydiol\ (I)) + n(Oxydicarbonsäure\ (II))] \ \leq 0{,}019$$

wobei "n(Pt)" für die Molmenge an Platin, "n(Oxydiol (I))" für die Molmenge an Oxydiol (I) und "n(Oxydicarbonsäure (II))" für die Molmenge an Oxydicarbonsäure (II) steht. Das genannte Molverhältnis beträgt bevorzugt ≥ 0,005 und besonders bevorzugt ≥ 0,007, sowie bevorzugt ≤ 0,017 und besonders bevorzugt ≤ 0,015.

In diesem Zusammenhang sei jedoch auch erwähnt, dass neben der beabsichtigten Oxidation der $-CH_2OH$ Gruppen zu den entsprechenden -COOH Gruppen gemäß dem dargestellten Reaktionsschema

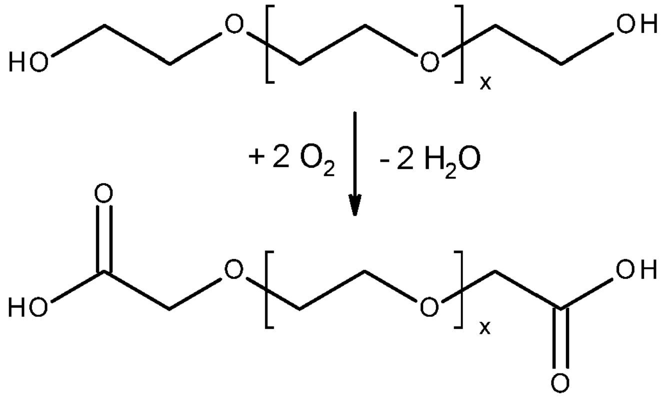


,

wobei x für eine positive ganze Zahl von 1 bis 10 steht, auch im geringen Umfang ein oxidativer Abbau von $-CH_2CH_2O-$ Gruppen stattfindet. Für ein Oxydiol (I) mit x gleich 1 bis 10 ist die exemplarische Reaktionsgleichung für den oxidativen Abbau nachfolgend dargestellt.

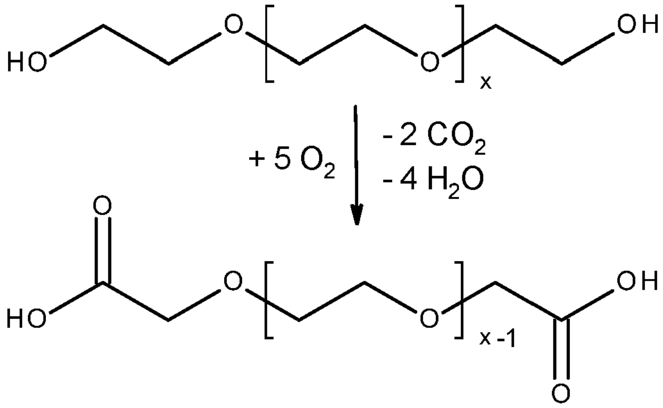


Die exemplarische Reaktionsgleichung für die Bildung von Glykolsäure geht von Oxydiol (I) mit x=0 aus.

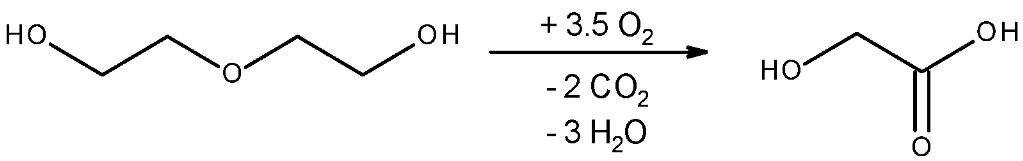


Durch den, im geringen Umfang stattfindenden oxidativen Abbau nimmt beim erfindungsgemäßen Verfahren die mittlere Kettenlänge etwas ab, so dass diese bei der erhaltenen Oxydicarbonsäure (II) etwas niedriger ist als jene beim eingesetzten Oxydiol (I). Allerdings ist die Abnahme der genannten mittleren Kettenlänge wesentlich kleiner als jene bei einem Verfahren oberhalb des erfindungsgemäßen Verhältnisses der Molmenge an Platin bezogen auf die Summe der Molmengen an Oxydiol (I) und Oxydicarbonsäure (II).

Die hohe Ausbeute an Oxydicarbonsäure (II) bei gleichzeitig sehr niedriger Bildung von Glykolsäure wird beim erfindungsgemäßen Verfahren ohne Zusatz basischer Verbindungen erreicht. Entsprechend führt man die Oxidation

bei einem pH-Wert von 1 bis 7 durch. Da der Einsatzstoff Oxydiol (I) in wässriger Lösung pH-neutral ist, liegt der pH-Wert zu Beginn der Oxidation üblicherweise bei oder nahe 7. Durch die Bildung der Oxydicarbonsäure (II) sinkt der pH-Wert allmählich, so dass gegen Ende der Oxidation in der Regel ein Wert von 1 bis 2 vorliegt.

Durch die Abwesenheit basischer Verbindungen bildet sich direkt die gewünschte Oxydicarbonsäure (II) und nicht dessen Salz. Dies hat den enormen Vorteil, dass die gewünschte Oxydicarbonsäure (II) anschließend nicht erst durch Zugabe einer stöchiometrischen Menge an Fremdsäure freigesetzt werden muss. Dies erspart insgesamt (i) den Einsatz zusätzlicher Chemikalien (Base und Fremdsäure), (ii) einen nachfolgenden Aufarbeitungsschritt samt Isolierung der Oxydicarbonsäure (II), und (iii) die Entsorgung des gebildeten Salzes aus der Base und der Fremdsäure.

Als Oxidationsmedium wird beim erfindungsgemäßen Verfahren molekularer Sauerstoff eingesetzt. Sauerstoff wird dabei entweder in reiner Form oder verdünnt mit anderen Gasen, beispielsweise in Form von Luft oder einem $O_2/N_2$-Gemisch zugegeben. Um bei einem vorgegebenen Sauerstoff-Partialdruck die Menge an einzusetzendem Sauerstoff so gering wie möglich zu halten, ist der Einsatz eines Gases mit möglichst hohem Sauerstoff-Gehalt vorteilhaft. Bevorzugt setzt man daher ein sauerstoffhaltiges Gas mit einem Gehalt von ≥ 90 Vol.-%, besonders bevorzugt von ≥ 95 Vol.-%, ganz besonders bevorzugt von ≥99 Vol.-% und insbesondere von ≥ 99,5 Vol.-% ein. Durch den Einsatz von möglichst hoch konzentrierten beziehungsweise reinen Sauerstoff ist es möglich, die Menge an Abgas relativ gering zu halten.

Um die Verteilung des Sauerstoffs im Reaktor zu begünstigen, ist es gegebenenfalls vorteilhaft, diesen feinperlig, beispielsweise durch eine Fritte, zuzudosieren.

Der bei der Oxidation vorliegende Sauerstoff-Partialdruck beträgt 0,01 bis 2 MPa, bevorzugt ≥ 0,02 MPa und besonders bevorzugt ≥ 0,05 MPa, sowie bevorzugt ≤ 1 MPa und besonders bevorzugt ≤ 0,3 MPa.

Die Oxidation erfolgt bei einer Temperatur von 20 bis 100° C, bevorzugt ≥ 30° C und besonders bevorzugt ≥ 40° C, sowie bevorzugt ≤ 80° C und besonders bevorzugt ≤ 70° C.

Als Reaktoren zur Durchführung des erfindungsgemäßen Verfahrens eignen sich prinzipiell alle Reaktoren, die für die Durchführung exothermer gas/flüssig-Reaktionen geeignet sind. So seien beispielhaft Rührkessel, Rieselbettreaktoren und Blasensäulenreaktoren genannt. Um die Reaktionswärme abzuführen sind die Reaktoren üblicherweise mit einer Kühleinrichtung ausgestattet. Je nach Reaktortyp und Art des Katalysators handelt es sich dabei vorteilhafterweise um innerhalb des Reaktors liegende Kühlelemente oder um außerhalb des Reaktors in einem externen Kreislauf vorhandene Kühlelemente. So enthält beispielsweise ein Rührkessel bevorzugt innenliegende Kühlelemente, wohingegen es beispielsweise bei einer Blasensäule vorteilhafter ist, die Kühlelemente im externen Kreislauf zu integrieren.

Liegt der Katalysator als Formkörper vor, so wird dieser üblicherweise in Form eines Festbettes im Reaktor fixiert. Hierzu bietet sich besonders der Rieselbettreaktor an, in dem der Katalysator in Form einer Schüttung eingebracht werden kann. Es ist aber möglich, Katalysatorformkörper in einem Rührkesselreaktor einzusetzen. In diesem Fall ist es dann vorteilhaft, die Katalysatorformkörper in einem Kompartiment, beispielweise einem Drahtkorb, zu fixieren.

Bei dem bevorzugten Einsatz eines pulverförmigen Katalysators liegt dieser vorteilhafterweise im Reaktionsgemisch suspendiert vor. Bevorzugte Reaktoren hierfür sind beispielsweise Rührkessel oder Blasensäule. Um ein Absetzen des pulverförmigen Katalysators zu vermeiden, ist eine entsprechende Durchmischung des flüssigen Reaktionsgemischs erforderlich. Diese wird in einem Rührkessel üblicherweise durch Einsatz eines Rührers erreicht. Im Falle einer Blasensäule wird die Vermischung gewöhnlich durch einen externen Kreislauf mit Förderpumpe erreicht. Prinzipiell kann die Blasensäule hinsichtlich des Flüssigkreislaufs zwar sowohl in Aufwärtsrichtung als auch in Abwärtsrichtung betrieben werden, jedoch ist der Betrieb in Abwärtsrichtung üblicherweise vorteilhafter.

Beim erfindungsgemäßen Verfahren kann sowohl halbkontinuierlich als auch kontinuierlich betrieben werden. In beiden Fällen wird der Sauerstoff zur Sicherstellung des gewünschten Partialdrucks kontinuierlich oder zumindest intermittierend, bevorzugt jedoch kontinuierlich dem Reaktor zudosiert.

Bei der halbkontinuierlichen Fahrweise wird vor Beginn der Reaktion die komplette Menge an wässrigem Eduktgemisch samt Katalysator im Reaktor vorgelegt und während der Oxidationsreaktion weder frisches Edukt zugeführt noch flüssiges Reaktionsgemisch entnommen. Eine Entleerung des Reaktors erfolgt erst nach Ende der Oxidationsreaktion.

Bei der kontinuierlichen Fahrweise befindet sich zwar ebenfalls flüssiges Reaktionsgemisch samt Katalysator im Reaktor, es wird jedoch stets eine kleine Menge an flüssigem Reaktionsgemisch entnommen und eine entsprechende Menge an wässrigem Edukt zugeführt. Falls hierbei ein Suspensionskatalysator eingesetzt wurde, wird das flüssige Reaktionsgemisch vorteilhafterweise über eine Filtereinrichtung, beispielsweise einem Querstromfilter, aus dem Reaktor entnommen.

Beim erfindungsgemäßen Verfahren ist es natürlich erstrebenswert einen möglichst hohen Umsatz an Oxydiol (I) zu erreichen. Da die Reaktionsgeschwindigkeit im Wesentlichen durch die Temperatur, den Sauerstoff-Partialdruck sowie die Art des heterogenen Katalysators vorgegeben ist, wird der gewünschte Umsatz bei den vorgegebenen Rahmenbedingungen schließlich durch die Reaktionsdauer eingestellt. Bevorzugt wählt man eine Reaktionsdauer, die einen Umsatz an Oxydiol (I) von 90 bis 100%, besonders bevorzugt von 95 bis 100%, ganz besonders bevorzugt von 99 bis

100% und insbesondere von 99,9 bis 100% ermöglicht. Üblicherweise liegt die Reaktionsdauer zur Erreichung eines Umsatzes an Oxydiol (I) von 90 bis 100% bei 10 bis 60 Stunden. Die im konkreten Einzelfall erforderliche Reaktionsdauer wird vorteilhafterweise entweder durch Vorversuche unter den entsprechenden Reaktionsbedingungen oder durch die Aufnahme geeigneter Messwerte während der Reaktionsführung ermittelt. Als geeignete Messwerte seien physikalische oder chemische Analysen des aktuell vorliegenden Reaktionsgemisches oder die Menge an verbrauchtem Sauerstoff genannt. Die Durchführung physikalischer Analysen kann beispielsweise offline durch Probenentnahme oder online im Reaktor oder in einem externen Kreislauf erfolgen. Als mögliche physikalische Methoden seien die Gaschromatographie sowie die Messung der elektrischen Leitfähigkeit, der Dielektrizitätskonstante oder der Impedanz genannt. Im Falle der Messung der Menge an verbrauchtem Sauerstoff wird in der Regel die Menge an zugeführtem Sauerstoff herangezogen.

Nach dem Ende der Umsetzung wird das Reaktionsgemisch üblicherweise aus dem Reaktor entnommen und vom Katalysator getrennt. Im Falle des Einsatzes eines Suspensionskatalysators erfolgt die Abtrennung sinnvollerweise durch Filtration. Alternativ ist es aber auch möglich, den Suspensionskatalysator nach dem Ende der Reaktion am Reaktorboden absetzen zu lassen und die überstehende Flüssigkeit zu entnehmen. Der abgetrennte Katalysator kann in der Regel ohne weitere Aufarbeitung wiederverwendet werden.

Das durch das erfindungsgemäße Verfahren erhältliche Reaktionsgemisch zeichnet sich durch einen relativ geringen Gehalt an unerwünschter Glykolsäure aus. Dieser beträgt üblicherweise nur 0 bis 1 Gew.-%, bevorzugt ≥ 0,1 Gew.-% und besonders bevorzugt ≥ 0,2 Gew.-%, sowie bevorzugt ≤ 0,7 Gew.-% und besonders bevorzugt ≤ 0,5 Gew.-%, bezogen auf die Oxydicarbonsäure (II).

Da das vom Katalysator abgetrennte Reaktionsgemisch einen erheblichen Teil Wasser enthält, ist es vor dessen Einsatz als Korrosionsschutzkomponente für ein Gefrierschutzmittel vorteilhaft, zumindest einen Teil des Wassers zu entfernen. Die technisch einfachste Möglichkeit ist hierbei die destillative Entfernung, da der Siedepunkt von Wasser deutlich unter den Siedepunkten der Oxydicarbonsäuren (II) liegt. Daher ist es beim erfindungsgemäßen Verfahren bevorzugt, aus dem erhaltenen Reaktionsgemisch Wasser destillativ zu entfernen. Vorteilhafterweise führt man die destillative Entfernung von Wasser im Vakuum, bevorzugt bei einem Druck von 0,001 bis 0,01 MPa abs durch.

Das durch destillative Entfernung von Wasser aufbereitete Reaktionsgemisch weist bevorzugt einen Wassergehalt von 0 bis 40 Gew.-%, bevorzugt von ≥ 1 Gew.-% und besonders bevorzugt von ≥ 2 Gew.-%, sowie bevorzugt von ≤ 30 Gew.-%, besonders bevorzugt von ≤ 20 Gew.-% und ganz besonders bevorzugt von ≤ 10 Gew.-% auf.

Die durch das erfindungsgemäße Verfahren hergestellte Oxydicarbonsäure (II) kann hervorragend als Korrosionsschutzkomponente für ein Gefrierschutzmittel eingesetzt werden.

In einer allgemeinen Ausführungsform des erfindungsgemäßen Verfahrens legt man die gewünschte Menge an Oxydiol (I) in einem Rührkessel zusammen mit der gewünschten Menge an pulverförmigen Pt/Aktivkohle-Katalysator vor und bringt den Inhalt unter Rühren auf die gewünschte Reaktionstemperatur. Nun beginnt man unter weiterem Rühren mit der Einleitung von Sauerstoff und stellt über eine Druckhaltung den gewünschten Gesamtdruck ein. Den Verlauf der Oxidation ermittelt man im einfachsten Falle aus der Menge an zudosiertem Sauerstoff. Nach Erreichen des gewünschten Umsatzes stoppt man die Zufuhr von Sauerstoff, kühlt ab, entspannt und entnimmt das erhaltene Reaktionsgemisch unter Abfiltration des Katalysators. Das vom Katalysator befreite Reaktionsgemisch wird dann in einer bevorzugten Variante im Vakuum von der Hauptmenge des vorhandenen Wassers befreit.

Das derart aufbereitete Gemisch kann dann problemlos als Korrosionsschutzkomponente für ein Gefrierschutzmittel eingesetzt werden. In der weiteren Formulierung kann man dann die weiteren Korrosionsschutz- und Gefrierschutz-Komponenten zumischen.

Das erfindungsgemäße Verfahren ermöglicht die Herstellung einer, für den Einsatz als Korrosionsschutzkomponente für ein Gefrierschutzmittel geeigneten Oxydicarbonsäure in hoher Ausbeute und Reinheit. Das als Einsatzstoff einzusetzende Oxydiol ist großtechnisch einfach herzustellen und somit auch in größeren Mengen gut verfügbar. Das erfindungsgemäße Verfahren zeichnet sich weiterhin dadurch aus, dass der Gehalt an Glykolsäure im Reaktionsprodukt relativ niedrig ist und somit eine aufwendige Aufreinigung entfällt. Durch eine simple Entwässerung im Vakuum kann ein wasserarmes Produktgemisch erhalten werden.

## Beispiele

### Gaschromatographische Analyse

Das in den Beispielen eingesetzt Oxydiol (I) sowie das erhaltene Reaktionsprodukt wurden jeweils gaschromatographisch hinsichtlich seiner organischen Komponenten analysiert. Hierzu wurde wie folgt vorgegangen:

| | |
|---|---|
| Gaschromatograph: | Agilent 7890B |
| Säule: | Rxi-1ms (30 m Länge, 0.25 mm (ID), 0.25 μm (Film) |
| Temperaturprogramm: | 3 Minuten bei 60° C, Aufheizen von 60° C auf 290° C mit 5° C/min, 12 Minuten bei 290° C |

(fortgesetzt)

| | |
|---|---|
| Probenvorbereitung: | Der Katalysator wurde abfiltriert und das Wasser entfernt. 50 mg der wasserfreien Mischung wurden dann mit 1 mL MSTFA (N-Methyl-N-(trimethylsilyl)-trifluoroacetamid) vermischt, für 1 Stunde auf 80° C erhitzt und die Probe in den Gaschromatographen injiziert. |

Beispiel 1 (Vergleichsbeispiel)

200 g pulverförmiger Katalysator mit 5 Gew.-% Platin auf Aktivkohle, entsprechend 10 g beziehungsweise 0,0513 mol Pt (Bezugsquelle Sigma-Aldrich) wurden in einen 4-Liter Glasreaktor gefüllt und mit 957 g Wasser bei 1000 U/min aufgerührt. Anschließend wurden 410 g Oxydiol (I) mit der in Tabelle 1a dargestellten Verteilung und einer mittleren Molmasse von 200 g/mol zugegeben, auf 60° C thermostatisiert und unter weiterem Rühren 50 l/h Sauerstoff durch das Reaktionsgemisch geleitet. Das Molverhältnis von Pt zu Oxydiol (I) betrug somit 0,025, und die Konzentration an Wasser in der flüssigen Phase 70 Gew.-%. Da keine Base zugegeben wurde, lag der anfängliche pH-Wert bei 6,9. Nach 27 Stunden war Vollumsatz erreicht. Die Zufuhr an Sauerstoff wurde beendet, das Reaktionsgemisch abgekühlt und vom Glasreaktor abgelassen. Das Reaktionsgemisch hatte einen pH-Wert von 1,5. Über eine D4-Glasfilternutsche wurde es filtriert und der Filterkuchen dreimal mit jeweils 200 ml warmem Wasser gewaschen. Das Filtrat wurde dann am Rotationsverdampfer bei 45° C bei einem Druck bis 10 mbar eingeengt. Es wurden 280 g Produktgemisch mit der in Tabelle 1b dargestellten Zusammensetzung erhalten. Die Analysen der organischen Komponenten erfolgten jeweils gaschromatographisch. Der Wassergehalt wurde durch Titration nach Karl Fischer bestimmt.

Tabelle 1a (Edukt)

| Oxydiol (I) | x=0 | x=1 | x=2 | x=3 | x=4 | x=5 | x=6 | x=7 |
|---|---|---|---|---|---|---|---|---|
| GC Flächen% | 4,9 | 23,9 | 31,0 | 22,1 | 11,2 | 4,5 | 1,4 | 0,3 |

Tabelle 1b (Produkt)

| Oxydicarbonsäure (II) [GC-Flächen%] | y=0 | y=1 | y=2 | y=3 | y=4 |
|---|---|---|---|---|---|
| | 26,6 | 31,1 | 24,7 | 11,2 | 1,9 |
| Glykolsäure [GC-Flächen%] | 4,5 | | | | |
| Wasser [Gew.-%] | 7 | | | | |

Unter Berücksichtigung des Wassergehalts von 7 Gew.-% und unter der näherungsweisen Abschätzung, dass die 4,5 GC-Flächen% Glykolsäure etwa 4,5 Gew.-% Glykolsäure bezogen auf das wasserfreie Produktgemisch entsprechen, ergab sich somit eine Ausbeute von etwa 249 g Oxydicarbonsäure (II).

Beispiel 2 (erfindungsgemäß)

78 g pulverförmiger Katalysator des gleichen Typs wie in Beispiel 1 mit 5 Gew.-% Platin auf Aktivkohle, entsprechend 3,9 g beziehungsweise 0,020 mol Pt (Bezugsquelle Sigma-Aldrich) wurden in einen 4-Liter Glasreaktor gefüllt und mit 957 g Wasser bei 1000 U/min aufgerührt. Anschließend wurden analog Beispiel 1 410 g Oxydiol (I) mit der in Tabelle 1a dargestellten Verteilung und einer mittleren Molmasse von 200 g/mol zugegeben, auf 60° C thermostatisiert und unter weiterem Rühren 50 l/h Sauerstoff durch das Reaktionsgemisch geleitet. Das Molverhältnis von Pt zu Oxydiol (I) betrug somit 0,0098, und die Konzentration an Wasser in der flüssigen Phase 70 Gew.-%. Da keine Base zugegeben wurde, lag der anfängliche pH-Wert bei 6,9. Nach 67 Stunden war Vollumsatz erreicht. Die Zufuhr an Sauerstoff wurde beendet, das Reaktionsgemisch abgekühlt und vom Glasreaktor abgelassen. Das Reaktionsgemisch hatte ebenfalls einen pH-Wert von 1,5. Über eine D4-Glasfilternutsche wurde es filtriert und der Filterkuchen dreimal mit jeweils 200 ml warmem Wasser gewaschen. Das Filtrat wurde dann am Rotationsverdampfer bei 45° C bei einem Druck bis 10 mbar eingeengt. Es wurden 436 g Produktgemisch mit der in Tabelle 2b dargestellten Zusammensetzung erhalten. Die Analysen der organischen Komponenten erfolgten jeweils gaschromatographisch. Der Wassergehalt wurde durch Titration nach Karl Fischer bestimmt.

Tabelle 2b (Produkt)

| Oxydicarbonsäure (II) [GC-Flächen%] | y=0 | y=1 | y=2 | y=3 | y=4 |
|---|---|---|---|---|---|
| | 12,3 | 29,2 | 32,5 | 19,6 | 5,8 |
| Glykolsäure [GC-Flächen%] | 0,3 | | | | |
| Wasser [Gew.-%] | 4,9 | | | | |

Unter Berücksichtigung des Wassergehalts von 4,9 Gew.-% und unter der näherungsweisen Abschätzung, dass die 0,3 GC-Flächen% Glykolsäure etwa 0,3 Gew.-% Glykolsäure bezogen auf das wasserfreie Produktgemisch entsprechen, ergab sich somit eine Ausbeute von etwa 413 g Oxydicarbonsäure (II).

Die beiden Beispiele zeigen, dass bei einem erfindungsgemäßen Molverhältnis von Pt zu Oxydiol (I) von 0,0098 (Beispiel 2) eine um rund 65% höhere Ausbeute an Oxydicarbonsäure (II) erhalten wird als bei einem Molverhältnis von 0,025 (Beispiel 1). Zudem wurden bei dem erfindungsgemäßen Molverhältnis von Beispiel 2 nur eine äußerst geringe Menge von 0,3 GC-Flächen% an störender Glykolsäure gebildet, wohingegen bei dem höheren Verhältnis in Beispiel 1 mit 4,5 GC-Flächen% eine 15-mal höhere Menge an Glykolsäure erhalten wurde.

Beispiel 3

Der in Beispiel 2 abgetrennte Katalysator wurde erneut unter den in Beispiel 2 beschriebenen Versuchsbedingungen eingesetzt. Es wurden 464 g Produktgemisch mit der in Tabelle 3b dargestellten Zusammensetzung erhalten.

Tabelle 3b (Produkt)

| Oxydicarbonsäure (II) [GC-Flächen%] | y=0 | y=1 | y=2 | y=3 | y=4 |
|---|---|---|---|---|---|
| | 11,0 | 29,0 | 33,0 | 20,0 | 6,0 |
| Glykolsäure [GC-Flächen%] | 0,8 | | | | |
| Wasser [Gew.-%] | 6,8 | | | | |

Unter Berücksichtigung des Wassergehalts von 6,8 Gew.-% und unter der näherungsweisen Abschätzung, dass die 0,8 GC-Flächen% Glykolsäure etwa 0,8 Gew.-% Glykolsäure bezogen auf das wasserfreie Produktgemisch entsprechen, ergab sich somit eine Ausbeute von etwa 429 g Oxydicarbonsäure (II).

Beispiel 4

Der in Beispiel 3 abgetrennte Katalysator wurde erneut unter den in Beispiel 2 beschriebenen Versuchsbedingungen eingesetzt. Es wurden 467 g Produktgemisch mit der in Tabelle 4b dargestellten Zusammensetzung erhalten.

Tabelle 4b (Produkt)

| Oxydicarbonsäure (II) [GC-Flächen%] | y=0 | y=1 | y=2 | y=3 | y=4 |
|---|---|---|---|---|---|
| | 11,1 | 28,5 | 33,0 | 20,5 | 6,3 |
| Glykolsäure [GC-Flächen%] | 0,7 | | | | |
| Wasser [Gew.-%] | 7,3 | | | | |

Unter Berücksichtigung des Wassergehalts von 7,3 Gew.-% und unter der näherungsweisen Abschätzung, dass die 0,7 GC-Flächen% Glykolsäure etwa 0,7 Gew.-% Glykolsäure bezogen auf das wasserfreie Produktgemisch entsprechen, ergab sich somit eine Ausbeute von etwa 428 g Oxydicarbonsäure (II).

Die Beispiele 3 und 4 zeigen, dass der verwendete Katalysator mehrfach wiederverwendet werden kann.

## Patentansprüche

1. Verfahren zur Herstellung einer Korrosionsschutzkomponente für ein Gefrierschutzmittel durch Oxidation eines Oxydiols der allgemeinen Formel (I)

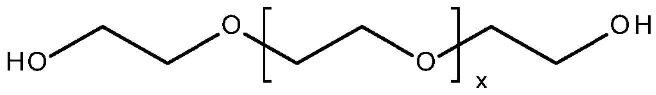

(I),

in der x für eine positive ganze Zahl von 1 bis 10 steht, mit molekularem Sauerstoff bei einer Temperatur von 20 bis 100°C und einem Sauerstoff-Partialdruck von 0,01 bis 2 MPa in Gegenwart von Wasser und eines heterogenen Katalysators, welcher Platin enthält, unter Bildung einer Oxydicarbonsäure der allgemeinen Formel (II)

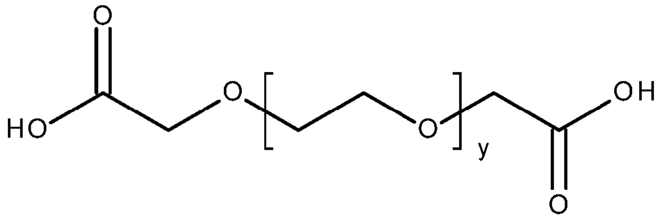

(II),

in der y für eine positive ganze Zahl von 1 bis 10 steht, **dadurch gekennzeichnet, dass** man die Oxidation

(a) bei einem Molverhältnis von

$$0,002 \leq n(Pt) / [n(Oxydiol (I)) + n(Oxydicarbonsäure (II))] \leq 0,019$$

wobei "n(Pt)" für die Molmenge an Platin, "n(Oxydiol (I))" für die Molmenge an Oxydiol (I) und "n(Oxydicarbonsäure (II))" für die Molmenge an Oxydicarbonsäure (II) steht;
(b) bei einer Konzentration an Wasser von 50 bis 95 Gew.-% in der flüssigen Phase; und
(c) bei einem pH-Wert von 1 bis 7

durchführt.

**2.** Verfahren nach Anspruch 1, **dadurch gekennzeichnet, dass** man ein Oxydiol (I) einsetzt, bei dem x für eine positive ganze Zahl von 1 bis 5 steht.

**3.** Verfahren nach den Ansprüchen 1 bis 2, **dadurch gekennzeichnet, dass** man ein Gemisch von Oxydiolen (I) mit einer mittlere Molmasse von 150 bis 300 g/mol einsetzt.

**4.** Verfahren nach den Ansprüchen 1 bis 3, **dadurch gekennzeichnet, dass** man einen heterogenen Katalysator enthaltend 0,1 bis 10 Gew.-% Platin auf Kohle einsetzt.

**5.** Verfahren nach den Ansprüchen 1 bis 4, **dadurch gekennzeichnet, dass** man einen heterogenen Katalysator einsetzt, welcher einen Gesamtgehalt an Cadmium, Blei und Bismut von 0 bis 0,1 Gew.-%, bezogen auf die Masse an Platin, aufweist.

**6.** Verfahren nach den Ansprüchen 1 bis 5, **dadurch gekennzeichnet, dass** man das Verfahren bei einem Molverhältnis von

$$0,005 \leq n(Pt) / [n(Oxydiol (I)) + n(Oxydicarbonsäure (II))] \leq 0,015$$

durchführt.

**7.** Verfahren nach den Ansprüchen 1 bis 6, **dadurch gekennzeichnet, dass** man ein Reaktionsgemisch mit einem Gehalt an Glykolsäure von 0 bis 1 Gew.-%, bezogen auf die Oxydicarbonsäure (II) erhält.

**8.** Verfahren nach den Ansprüchen 1 bis 7, **dadurch gekennzeichnet, dass** man aus dem erhaltenen Reaktionsgemisch Wasser destillativ entfernt.

9. Verfahren nach Anspruch 8, **dadurch gekennzeichnet, dass** man ein aufbereitetes Reaktionsgemisch mit einem Wassergehalt von 0 bis 40 Gew.-% erzeugt.

10. Verwendung einer gemäß den Ansprüchen 2 bis 3 hergestellten Oxydicarbonsäure (II) als Korrosionsschutzkomponente für ein Gefrierschutzmittel.

## Claims

1. A process for preparing an anticorrosion component for an antifreeze by oxidizing an oxydiol of the general formula (I)

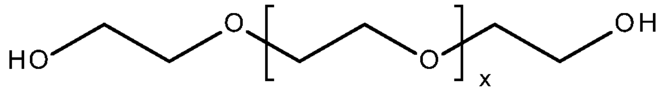

(I)

in which x is a positive integer from 1 to 10 with molecular oxygen at a temperature of 20 to 100°C and a partial oxygen pressure of 0.01 to 2 MPa in the presence of water and of a heterogeneous catalyst comprising platinum to form an oxydicarboxylic acid of the general formula (II)

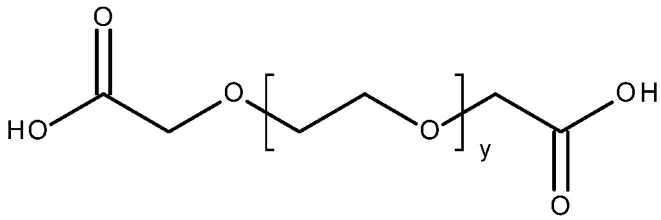

(II)

in which y is a positive integer from 1 to 10, which comprises conducting the oxidation

(a) at a molar ratio of

$$0.002 \leq n(\text{Pt}) / [n(\text{oxydiol (I)}) + n(\text{oxydicarboxylic acid (II)})] \leq 0.019$$

where "n(Pt)" is the molar amount of platinum, "n(oxydiol (I))" is the molar amount of oxydiol (I) and "n(oxydicarboxylic acid (II))" is the molar amount of oxydicarboxylic acid (II);
(b) at a concentration of water of 50% to 95% by weight in the liquid phase; and
(c) at a pH of 1 to 7.

2. The process according to claim 1, wherein an oxydiol (I) in which x is a positive integer from 1 to 5 is used.

3. The process according to claims 1 to 2, wherein a mixture of oxydiols (I) having an average molar mass of 150 to 300 g/mol is used.

4. The process according to claims 1 to 3, wherein a heterogeneous catalyst comprising 0.1% to 10% by weight of platinum on charcoal is used.

5. The process according to claims 1 to 4, wherein a heterogeneous catalyst having a total content of cadmium, lead and bismuth of 0% to 0.1% by weight, based on the mass of platinum, is used.

6. The process according to claims 1 to 5, wherein the process is conducted at a molar ratio of

$$0.005 \leq n(\text{Pt}) / [n(\text{oxydiol (I)}) + n(\text{oxydicarboxylic acid (II)})] \leq 0.015.$$

7. The process according to claims 1 to 6, wherein a reaction mixture having a content of glycolic acid of 0% to 1% by weight, based on the oxydicarboxylic acid (II), is obtained.

8. The process according to claims 1 to 7, wherein water is removed by distillation from the reaction mixture obtained.

9. The process according to claim 8, wherein a processed reaction mixture having a water content of 0% to 40% by weight is produced.

10. The use of an oxydicarboxylic acid (II) prepared according to claims 2 to 3 as anticorrosion component for an antifreeze.

**Revendications**

1. Procédé pour la préparation d'un composant de protection contre la corrosion pour un antigel par oxydation d'un oxydiol de formule générale (I)

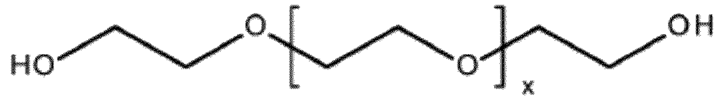

(I),

dans laquelle x représente un nombre entier positif de 1 à 10, avec de l'oxygène moléculaire à une température de 20 à 100 °C et à une pression partielle d'oxygène de 0,01 à 2 MPa en présence d'eau et d'un catalyseur hétérogène, lequel contient du platine, avec formation d'un acide oxydicarboxylique de formule générale (II)

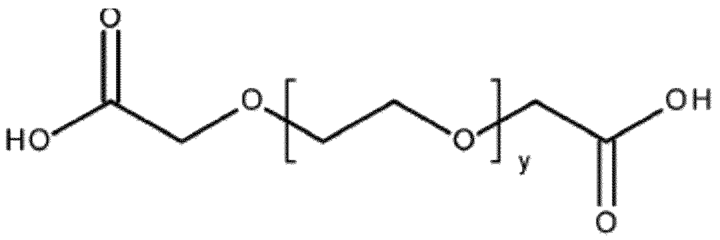

(II),

dans laquelle y représente un nombre entier positif de 1 à 10, **caractérisé en ce qu'**on met en œuvre l'oxydation

(a) à un rapport molaire de

$$0{,}002 \leq n(Pt) / [n(\text{oxydiol (I)}) + n(\text{acide oxydicarboxylique (II)})] \leq 0{,}019$$

« n(Pt) » représentant le nombre de moles de platine, « n(oxydiol(I)) » représentant le nombre de moles d'oxydiol (I) et « n(acide oxydicarboxylique (II)) » représentant le nombre de moles d'acide oxydicarboxylique (II) ;
(b) à une concentration en eau de 50 à 95 % en poids dans la phase liquide ; et
(c) à une valeur de pH de 1 à 7.

2. Procédé selon la revendication 1, **caractérisé en ce qu'**on utilise un oxydiol (I) dans lequel x représente un nombre entier positif de 1 à 5.

3. Procédé selon les revendications 1 et 2, **caractérisé en ce qu'**on utilise un mélange d'oxydiols (I) comportant une masse molaire moyenne de 150 à 300 g/mole.

4. Procédé selon les revendications 1 à 3, **caractérisé en ce qu'**on utilise un catalyseur hétérogène contenant 0,1 à 10 % en poids de platine sur charbon.

5. Procédé selon les revendications 1 à 4, **caractérisé en ce qu'**on utilise un catalyseur hétérogène, lequel présente une teneur totale en cadmium, en plomb et en bismuth de 0 à 0,1 % en poids, par rapport à la masse de platine.

6. Procédé selon les revendications 1 à 5, **caractérisé en ce qu'**on met en œuvre le procédé à un rapport molaire de

$$0{,}005 \leq n(\text{Pt}) / [n(\text{oxydiol (I)}) + n(\text{acide oxydicarboxylique (II)})] \leq 0{,}015.$$

7. Procédé selon les revendications 1 à 6, **caractérisé en ce qu'**on obtient un mélange réactionnel comportant une teneur en acide glycolique de 0 à 1 % en poids, par rapport à l'acide oxydicarboxylique (II).

8. Procédé selon les revendications 1 à 7, **caractérisé en ce qu'**on élimine de l'eau par distillation depuis le mélange réactionnel obtenu.

9. Procédé selon la revendication 8, **caractérisé en ce qu'**on génère un mélange réactionnel préparé comportant une teneur en eau de 0 à 40 % en poids.

10. Utilisation d'un acide oxydicarboxylique (II) préparé selon les revendications 2 et 3 en tant que composant de protection contre la corrosion pour un antigel.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0479470 B1
- EP 0552988 B1
- US 4561990 A
- EP 0229440 B1
- US 4238625 A
- DE 3135946 A1
- DE 3135846 A1
- DE 2936123 A
- US 4256916 A


### In der Beschreibung aufgeführte Nicht-Patentliteratur


- **Y. EIN-ELI.** *Electrochemical and Solid-State Letters,* 2004, vol. 7 (1), B5-B7
- **K.HEYNS et al.** *Tetrahedron,* 1960, vol. 9, 67-75
- **C.DONZE et al.** *Applied Catalysis B: Environmental,* 2007, vol. 70, 621-629
- **H.FIEGE et al.** *Angew. Chem.,* 1981, vol. 93 (9), 812-813